# EUROPEAN PATENT APPLICATION

(11) **EP 4 530 251 A1**
(43) Date of publication of application: **02.04.2025**
(21) Application number: 23200364.0
(22) Date of filing: 28.09.2023
(51) Int. Cl.: C01B 3/36, C01B 3/38, C01B 3/48, C01B 3/50

(54) **DECARBONISATION OF A CHEMICAL PLANT**

(71) Applicant: Johnson Matthey Public Limited Company, London EC2V 7AD (GB)
(72) Inventor: The designation of the inventor has not yet been filed
(74) Representative: Johnson Matthey Plc

(57) **Abstract**

The specification describes a chemical plant comprising an off-gas treatment unit arranged to accept a hydrocarbon-containing fuel stream comprising hydrocarbons, steam, and hydrogen, the off-gas treatment unit comprising:
(i) a hydrogen membrane separation unit arranged to accept the hydrocarbon-containing fuel stream and produce a permeate stream and a retentate stream;
(ii) a reforming section comprising a partial oxidation reactor or an autothermal reformer, arranged to accept the retentate stream and produce a reformed gas stream;
(iii) a water-gas shift section arranged to accept said reformed gas stream and produce a shifted gas stream;
(iv) a CO₂ removal section comprising one or more CO₂ removal units, the CO₂ removal section arranged to accept said shifted gas stream and produce a CO₂ rich stream and a hydrogen product stream;
wherein at least a portion of the permeate stream and/or the hydrogen product stream is used as a low carbon fuel.

Also described is a process for converting a hydrocarbon-containing fuel stream into a low carbon fuel which is carried out in an off-gas treatment unit as described. Also described is a retrofitting method for a chemical plant, in which an off-gas treatment unit as described is installed.

## Description

### Technical field

The present invention relates to a method for reducing the carbon dioxide emissions of a chemical plant and thus enabling a lower carbon intensity product from that plant, and potentially an increase in production capacity.

### Background art

Numerous chemical processes involve a step in which hydrocarbon-containing impurities are removed from the reaction system or desired product via a purge or a purification unit. This hydrocarbon-containing stream is often referred to as "off-gas", "fuel gas" or "purge gas". Important, non-limiting examples of this are in the production of hydrogen, methanol and ammonia; production of these chemicals industrially involves steam reforming of a hydrocarbon feed to produce a synthesis gas which is then processed in downstream steps. Unwanted hydrocarbons (predominantly methane) are removed via a reaction loop purge or via a purification unit as an off-gas. Normally the off-gas is combusted in a steam methane reformer (SMR) or one or more fired heaters as fuel for use elsewhere on the chemical plant. The CO₂ produced through burning the off-gas is not normally captured because it is at relatively low pressure. This CO₂ is therefore released to the atmosphere (sometimes referred to as flue gas) and contributes to the CO₂ emissions of the chemical plant and thus carbon intensity assigned to the desired product, e.g. H₂, methanol, ammonia, ethylene, etc.

WO2022/003312A1 describes a process for the production of hydrogen which involves: (i) steam reforming of a mixture comprising a hydrocarbon and steam at a steam-to-carbon ratio of at least 2.6 : 1 in a gas-heated reformer followed by autothermal reforming; (ii) water-gas shift; (iii) condensation of water; (iv) carbon dioxide separation; and (v) purification to separate a purified hydrogen gas and a fuel gas. The fuel gas is fed, as the sole fuel, to one or more fired heaters used to heat one or more process streams within the process.

WO2022/003313A1 describes a similar concept to WO2022/003312A1 but instead of step (i) as described above the process involves subjecting a gaseous mixture comprising hydrocarbon and steam having a steam to carbon ratio of at least 0.9 : 1 to adiabatic pre-reforming in a pre-reformer followed by autothermal reforming in an autothermal reformer (ATR).

WO2022/003312A1 and WO2022/003313A1 describe arrangements which are well suited to new build "grassroots" hydrogen plants. For the avoidance of doubt, a "hydrogen plant" is a chemical plant in which H₂ is the desired product, as opposed to simply an intermediate as is the case in a methanol plant or an ammonia plant. However, because the capex of a grassroots chemical plant is high, there is a need for solutions which decarbonise existing chemical plants.

One solution is to increase the yield of hydrogen of an existing process and use a portion of this hydrogen as fuel instead of the hydrocarbon-containing off-gas. For example WO2011/046680A1 discloses a method and apparatus for producing a hydrogen-containing product in which hydrocarbon-containing feed gas streams are reacted in a steam methane reformer of an existing hydrogen plant and a catalytic reactor that reacts hydrocarbons, oxygen and steam. The catalytic reactor is retrofitted to the existing hydrogen plant to increase hydrogen production. The resulting synthesis gas streams are combined, cooled, subjected to water-gas shift and then introduced into a production apparatus that can be a pressure swing adsorption unit. The amount of synthesis gas contained in a shifted stream made available to the production apparatus is increased by virtue of the combination of the synthesis gas streams to increase production of the hydrogen containing product. The catalytic reactor is operated such that the synthesis gas stream produced by such reactor is similar to that produced by the steam methane reformer and at a temperature that will reduce oxygen consumption within the catalytic reactor.

The above described retrofit process involves adding a catalytic reactor into the main steam reforming section. This necessarily involves disrupting the main reforming section requiring plant downtime and is complex and expensive.

The present invention provides an alternative solution to reducing the CO₂ emissions of a chemical plant and is applicable to a wide range of chemical plants where an off-gas is currently combusted as fuel.

### Summary of the invention

The present inventors have realised that the above problems can be solved by retrofitting the chemical plant by installing an off-gas treatment unit (OTU). The role of the OTU is to convert a hydrocarbon-containing fuel stream into a low carbon fuel which can be combusted instead of a hydrocarbon fuel.

In a first aspect the invention relates to a chemical plant comprising an off-gas treatment unit arranged to accept a hydrocarbon-containing fuel stream comprising hydrocarbons, steam, and hydrogen, the off-gas treatment unit comprising:
(i) a hydrogen membrane separation unit arranged to accept the hydrocarbon-containing fuel stream and produce a permeate stream and a retentate stream;
(ii) a reforming section comprising a partial oxidation reactor or an autothermal reformer, arranged to accept the retentate stream and produce a reformed gas stream;
(iii) a water-gas shift section arranged to accept said reformed gas stream and produce a shifted gas stream;
(iv) a CO₂ removal section comprising one or more CO₂ removal units, the CO₂ removal section arranged to accept said shifted gas stream and produce a CO₂ rich stream and a hydrogen product stream;
wherein at least a portion of the permeate stream and/or the hydrogen product stream is used as a low carbon fuel.

The H₂ membrane separation unit is located upstream of the partial oxidation (POX) reactor or ATR. This arrangement is advantageous where the feed stream to the reforming section has a significant hydrogen content because it avoids wastage of the hydrogen in the POX reactor or ATR. A purge stream from a methanol loop typically contains significant amounts of hydrogen along with unreformed methane and any inerts such as N₂, and therefore in a preferred embodiment the hydrocarbon-containing fuel stream is produced using a purge stream from a methanol loop.

In addition to the H₂ membrane separation unit located upstream of the POX reactor or ATR, a H₂ membrane separation unit may also be located downstream from the water-gas shift section and upstream from the CO₂ removal section within the OTU. The role of this membrane separation unit is to remove hydrogen produced during the water-gas shift stage(s). The introduction of a H₂ membrane in between stages of water-gas shift and CO₂ removal is described in WO2011/026943A1.

The benefit of introducing a H₂ membrane separation unit at these location is twofold. Firstly, by removing H₂ upstream of the reforming section, volume flow to downstream operations is reduced thus making the equipment smaller which is preferable from a capex and opex perspective. Secondly, when a H₂ membrane separation unit is located downstream from the water-gas shift section and upstream from the CO₂ removal section, the CO₂ partial pressure of the retentate fuel is increased making CO₂ adsorption more efficient which is preferable from a capex and opex perspective.

When applied to the decarbonisation of chemical plants containing a reforming section, as is found in many hydrogen, methanol and ammonia plants and ethylene and naphtha reformers for aromatics production, the option of fitting the OTU outside of the main reforming section offers several benefits. Firstly, it does not involve disrupting the main reforming section which is complex, expensive and requires plant downtime. Secondly, a single OTU, located wherever is most convenient and within practical pipeline distance of the plant(s) being decarbonised, can be used for the processing of several different off-gas stream from different locations throughout the plant. This option is therefore particularly attractive for a chemical plant or chemical manufacturing site, e.g. an oil refinery or petrochemicals complex, where there are several different hydrocarbon-containing off-gas streams which are used for fuel.

This solution is different from the solution in WO2011/046680A1 where a catalytic reactor is retrofitted directly between the steam methane reformer and the WGS section in the main reforming section. In the prior art arrangement, off-gases from elsewhere cannot easily be sent to the reforming operations. The present invention offers the advantage that, by having a dedicated OTU, it is possible to send hydrocarbon-containing off-gas streams from multiple different sources throughout the chemical plant for treatment, and generate a low carbon fuel for fired equipment. For the avoidance of doubt, the low carbon fuel stream may contain some residual carbon containing compounds, depending upon practical extent of decarbonisation when overall capex and opex are considered.

In a second aspect the invention relates to a process for converting a hydrocarbon-containing fuel stream into a low carbon fuel, wherein the process is carried out in an off-gas treatment unit as defined herein.

In a third aspect the invention relates to method for retrofitting a chemical plant, comprising the step of installing an off-gas treatment unit as defined herein.

Whilst arrangements according to the first aspect can be prepared by the retrofit method, the arrangement may also be useful for grassroots chemical plants.

### Description of the Figures

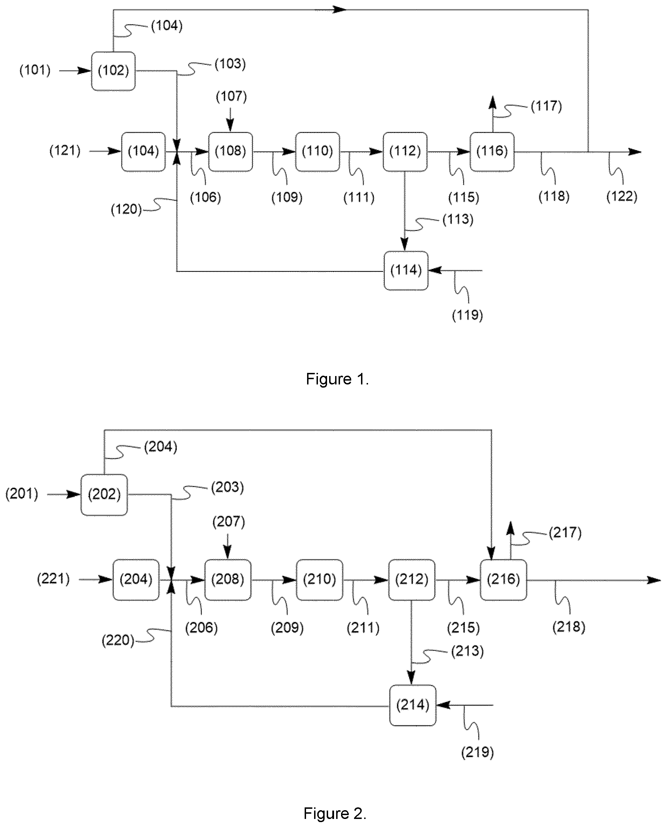

Figure 1 is an illustration of an off-gas treatment unit according to the present invention. A hydrocarbon-containing fuel stream (101), optionally having first undergone purification, is treated in a hydrogen membrane separation unit (102) to produce a retentate stream (103) which is relatively depleted in hydrogen and a permeate stream (104) which is relatively enriched in hydrogen. The retentate stream is combined with steam (120) to produce stream (106) and fed to an autothermal reformer (108). A supplementary fuel (121), typically having first undergone treatment in a purification unit (104), may also be added to produce stream (106). An oxygen-containing stream (107) is also fed to the autothermal reformer. A reformed gas stream (109) is fed to a water-gas shift section (110) which includes one or more shift units. A shifted gas stream (111) is fed to a cooling and water recovery unit (112) where it is separated into a water stream (113) and a crude H₂ stream (115). The crude H₂ stream is fed to a CO₂ separation section (116) where it is separated into a CO₂-rich stream (117) and a hydrogen product stream (118). The water stream (113), along with make-up water (119) is fed to a steam boiler (114) where it is used to generate steam (120) which is fed back to the process upstream of the autothermal reformer and/or upstream of water-gas shift. In the arrangement shown the permeate stream and hydrogen product stream are combined to produce a low carbon fuel stream (122).
Figure 2 is an illustration of an off-gas treatment unit according to the present invention. The arrangement is the same as for Figure 1 but the permeate pressure is such that the purity of the permeate stream (204) is low enough that it requires further CO₂ removal before being used as fuel. The permeate stream is fed to the CO₂ separation section (216). In the arrangement shown the CO₂ separation section produces a single CO₂-rich stream; in the case of a reactive wash system this may be achieve by sending CO₂-laden adsorbent to a common regenerator.

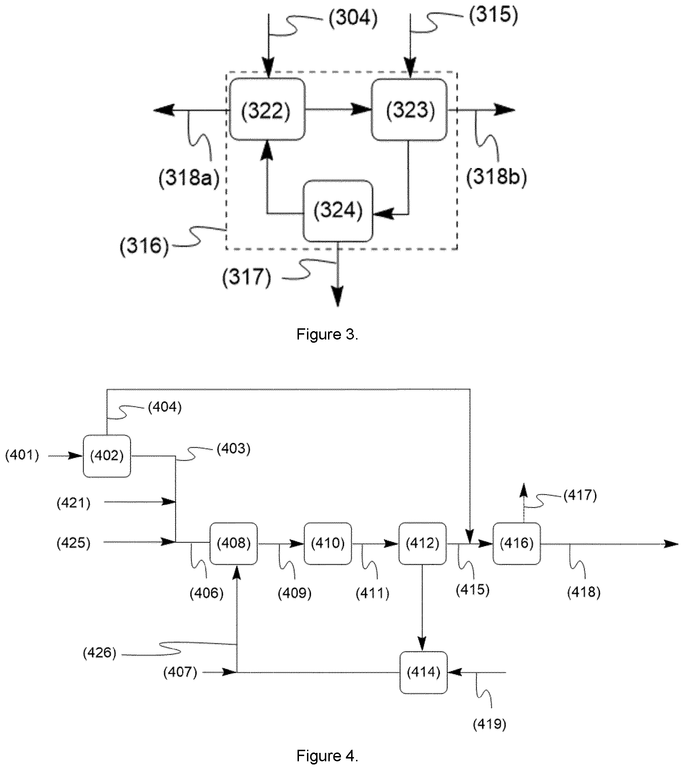

Figure 3 is an illustration of an arrangement of the CO₂ separation section (316) which accepts two streams at different pressures. The permeate stream (304) is fed to a permeate CO₂ absorption vessel (322) where CO₂ is removed to produce a first hydrogen product stream (318a). A crude H₂ stream (315) and CO₂-laden adsorbent produced by the permeate CO₂ absorption vessel are fed to a shifted gas CO₂ absorption vessel (323) where CO₂ is removed to produce a second hydrogen product stream (318b). The shifted gas CO₂ absorption vessel operates at a higher pressure than the permeate CO₂ absorption vessel. CO₂-laden adsorbent produced by the shifted gas absorption vessel is fed to a regeneration vessel (324) where it is separated into a CO₂-rich stream (317) and lean absorbent which is recycled to the permeate CO₂ absorption vessel. Streams (318a) and (318b) may be used separately or may be combined to produce a low carbon fuel stream.
Figure 4 is an illustration of a variant of the arrangement in Figure 2 with the following changes. Firstly, the permeate stream (404) and the crude H₂ stream (415) are combined and sent to the CO₂ separation section (416). Secondly, steam (420) generated by steam boiler (414) is combined with an oxygen-containing steam (407) to produce stream (426) which is fed to the ATR (408). The retentate stream (403) is combined with a compressed off-gas added as a supplementary fuel (421) then combined with a natural gas stream (425) and steam injection (not shown) to produce stream (406). Numbered lines and unit are otherwise as shown for Figure 2.

### Detailed description

Any sub-headings are for convenience only and are not intended to limit the invention.

### Chemical plant

The present invention is applicable to a wide variety of chemical plants which produce at least one hydrocarbon-containing off-gas stream.

In the present specification the term "hydrocarbon-containing fuel stream" is used to refer to the stream containing hydrocarbons, steam and hydrogen, which is fed to the H₂ membrane separation unit. The hydrocarbon-containing fuel stream is made up from one or more hydrocarbon-containing off-gas streams, which may optionally have first been treated to remove contaminants, and optional addition of steam to adjust the steam to carbon ratio.

The skilled person will be aware that many chemical processes operate as a loop; the product stream from a reactor is treated to separate out a stream containing the desired product and a stream containing unreacted materials which is recycled to the reactor. To avoid inerts building to unacceptably high levels in the loop a portion of the recycle stream is removed (a purge stream). In some embodiments the hydrocarbon-containing fuel stream is produced using one or more purge streams, for example a purge stream from a methanol loop.

In another embodiment a hydrocarbon-containing stream from a purification unit, e.g. a pressure-swing adsorption unit, is used to generate the hydrocarbon-containing fuel stream. In some embodiments the purification unit is a component of a steam reforming section comprising a steam methane reformer (e.g. in a hydrogen, methanol or ammonia plant).

In another embodiment a hydrocarbon-containing stream from an ethylene cracker is used to generate the hydrocarbon-containing fuel stream.

### Off-gas treatment unit

The role of the OTU is to convert the hydrocarbon-containing fuel stream into a low carbon fuel which can be used for heating duties. The low carbon fuel typically comprises ≥ 90 vol% H₂, such as ≥ 95 vol% H₂.

The hydrocarbon-containing fuel stream may be prepared from a single hydrocarbon-containing off-gas stream or by combining two or more different hydrocarbon-containing off-gas streams. The latter option has the benefit that the OTU can be used to decarbonise multiple different off-gas streams from across the chemical plant. Where multiple different off-gas streams are used to prepare the hydrocarbon-containing fuel stream, these will typically first be combined in a fuel gas header.

Depending on the composition of streams used to produce the hydrocarbon-containing fuel stream (e.g. one or more off-gas streams and any supplementary fuel), it may be necessary to carry out a step of pre-reforming on one or more of the streams. If one or more of the streams requires pre-reforming, then it is preferred that the streams used to produce the hydrocarbon-containing fuel stream are combined and sent to the pre-reformer to produce the produce the hydrocarbon-containing fuel stream, as this arrangement simplifies pre-heating of the feed to the reforming section.

Depending on the composition of the off-gas stream(s), it may be necessary to carry out a purification step before producing the hydrocarbon-containing fuel stream. Purification may include for instance H₂, Cl, Hg, sulfur removal, carbon monoxide removal, or CO₂ removal.

The combined energy available from combusting the permeate and hydrogen product streams is less than that available from combusting the streams used to produce the hydrocarbon-containing fuel stream. This may not be problematic in all cases, but in some cases supplementary fuel is needed to compensated for the shortfall. The supplementary fuel is typically a natural gas stream. If necessary, the supplementary fuel may first be treated in a purification unit. The supplementary fuel may be used to produce the hydrocarbon-containing fuel stream or may be added to the retentate stream upstream of the reforming section.

In addition to the hydrogen membrane separation unit the OTU comprises a reforming section, a water-gas shift section, and a CO₂ removal section. These are described in detail in subsequent sections. Additional units (e.g. heat exchangers, steam removal etc...) may also be present in the OTU.

### H₂ membrane separation unit

The design of membranes for the separation of H₂ from other gases will be known to those skilled in the art and the type of membrane used in the present invention is not particularly limited. Suitable H₂ membranes include those described in WO2011/026943A1.

The H₂ membrane separation unit generates a permeate stream and a retentate stream. The permeate stream is relatively rich in H₂. The retentate stream is relatively depleted in H₂ and is fed to the reforming section.

There is a trade-off between the degree of hydrogen recovery and the purity of the permeate stream. At high permeate pressures the amount of H₂ recovered from the hydrocarbon-containing fuel stream is low but the purity of H₂ in the permeate is high. At low permeate pressures the amount of H₂ recovered from the hydrocarbon-containing fuel stream is high but the purity of H₂ in the permeate is low.

In some embodiments the permeate stream may be used as a low carbon fuel directly without further purification, e.g. CO₂ depletion. This may be the case if the low carbon fuel is produced with sufficiently high purity (e.g. ≥95% H₂ such as ≥97% H₂). Permeates with lower purity (e.g. 90-95% H₂) may be used without further CO₂ removal if the permeate stream is combined with the hydrogen product stream from the CO₂ removal section to produce a low carbon fuel stream with a sufficiently low CO₂ content.

In some embodiments the permeate stream may require CO₂ removal before being used as a low carbon fuel, for instance if the permeate is produced with low purity (e.g. < 90% H₂) or required extent of decarbonisation is high. This may be achieved by sending the permeate to the CO₂ removal section. If the permeate and the shifted gas stream (or dewatered shifted gas stream) are of sufficiently similar pressures then it is preferred that the permeate and the shifted gas stream are combined before being sent to the CO₂ removal section. This has the benefit that the CO₂ removal section only requires a single CO₂ separation unit which is advantageous for reasons of capex and opex. If the permeate and the shifted gas stream (or dewatered shifted gas stream) are of sufficiently different pressures then it is preferred that the permeate stream and the shifted gas stream are fed to separate CO₂ absorption units within the CO₂ removal section, rather than adding the complexity, capex and opex for a permeate stream compressor.

### Reforming section

The reforming section comprises a partial oxidation reactor or an autothermal reformer to which the retentate stream is fed. In some arrangements the retentate stream may be combined with a supplementary fuel before being fed to the reforming section. Examples of supplementary stream(s) include natural gas or hydrocarbon-containing streams. Any supplementary feed stream(s) will typically first have undergone purification before being combined with the retentate stream. However, it is generally preferred that any supplementary fuel is added downstream of the hydrogen membrane separation unit.

One of the properties of the mixture within the reformer is the steam to carbon ratio, defined as the ratio of steam to carbon atoms present as hydrocarbon. For the avoidance of doubt, a mixture containing 75 mol% H₂O and 25 mol% CH₄ has a steam to carbon ratio of 3.0 : 1, a mixture containing 75 mol% H₂O, 10 mol% CO and 15 mol% CH₄ has a steam to carbon ratio of 5.0 : 1 and so on. A steam to carbon ratio of at least 2.0 : 1 is required to theoretically convert all of the carbon present in hydrocarbons into CO₂ and H₂. Steam to carbon ratios above 2.0 to 1 may be beneficial to ensure that sufficient steam is present for complete conversion of hydrocarbons in the mixture. Because of the energy cost of raising steam it is preferred that the steam to carbon ratio is not more than 3.5 : 1. It is preferred that the steam to carbon ratio of the mixture within the reformer is from 0.5 : 1 to 3.5 : 1, such as 1.0 : 1 to 3.0 : 1. Typically, rather than adding steam upstream or downstream of the hydrogen membrane separation unit, steam is added to the oxygen-containing stream fed to the POX reactor or ATR.

### Partial oxidation reactor (POX reactor) or autothermal reformer (A TR)

The role of the POX reactor or ATR is to convert hydrocarbons in the retentate stream into hydrogen and carbon oxides through steam reforming reactions.

The skilled person will be familiar with the design of autothermal reformers and detail of their arrangement and suitable catalysts are summarised in WO2022/003312A1. An ATR generally comprises a burner disposed at the top of the reformer, to which the retentate stream and an oxygen-containing gas are fed, a combustion zone beneath the burner through which a flame extends, and a fixed bed of particulate steam reforming catalyst disposed below the combustion zone. In autothermal reforming, the heat for the endothermic steam reforming reactions is therefore provided by combustion of a portion of hydrocarbon in the retentate stream. The retentate stream is typically fed to the top of the reformer and the oxygen-containing gas fed to the burner, mixing and combustion occur downstream of the burner generating a heated gas mixture the composition of which is brought to equilibrium as it passes through the steam reforming catalyst.

Partial oxidation reactors are known and typically comprise a vessel to which the feed and an oxygen-containing gas are fed via a burner, analogous to that used in an autothermal reformer, disposed above a reaction chamber in which the partial combustion reactions take place. Unlike an autothermal reformer, a catalyst is not present in the vessel. Air, oxygen-enriched air or O₂ may be used as the oxygen-containing gas.

The combustion temperature may be about 1300°C, or higher. Steam may be added to the feed and/or the oxygen containing gas to lower the combustion temperature and reduce soot formation. The idealised formula for this reaction applied to methane in the feed is as follows:

2 CH₄ + O₂ → 2 CO + 4 H₂

However, yields are below stoichiometric because part of the feed is fully combusted. It is therefore preferred that the OTU comprises an ATR.

In some embodiments the reforming section consists essentially of an autothermal reformer, by which it is meant that the reforming section does not include any other reforming units other than the ATR.

In some embodiments the reforming section additionally comprises a gas-heated reformer which is used in conjunction with the POX reactor or ATR. In a preferred arrangement the reforming section comprises an autothermal reformer and a gas-heated reformer. The skilled person will be familiar with the design of gas-heated reformers and detail of their arrangement and suitable catalysts are summarised in WO2022/003312A1. The POX reactor or ATR and gas-heated reformer may be arranged in series or in parallel. When arranged in series, the retentate stream is fed to catalyst filled tubes in the gas-heated reformer (tube-side) where steam reforming reactions take place to produce a partially reformed gas stream. The partially reformed gas stream is fed to the POX reactor or ATR where further steam reforming reactions take place to produce a reformed gas stream. The reformed gas stream is fed to the shell of the gas-heated reformer to provide heat required for steam reforming taking place in the gas-heated reformer. When arranged in parallel, a first portion of the retentate stream is fed to catalyst filled tubes in the gas-heated reformer (tube-side) where steam reforming reactions take place to produce a first partially reformed gas stream. A second portion of the retentate stream is fed to the POX reactor or ATR where steam reforming reactions take place to produce a second partially reformed gas stream. The first and second partially reformed gas streams are combined and used to provide heat required for steam reforming taking place in the gas-heated reformer.

Where an autothermal reformer is present, it is preferred that the WGS section includes a high-temperature shift vessel and the off-gas treatment unit is arranged such that the combined reforming feed (including the retentate and any purified additional hydrocarbon feed, e.g. natural gas) stream is pre-heated by heat exchange with a shifted gas stream generated by the high-temperature shift vessel. High-temperature shift is operated adiabatically in a shift vessel with inlet temperature in the range 300-400°C, preferably 320-360°C, typically over a bed of a reduced iron catalyst, such as chromia-promoted magnetite. Alternatively, a promoted zinc-aluminate catalyst may be used. This arrangement reduces or eliminates the need for additional fuel to pre-heat the retentate stream. In a preferred embodiment the shifted gases from the high-temperature shift vessel provide all of the heating duty for the retentate stream.

The stream exiting the reforming stage(s) is referred to herein as a reformed gas stream.

The reformed gas stream is typically cooled before being fed to the WGS section. Typically to reduce cost and complexity, saturated steam is raised. However, it is preferred that the outlet gases from the POX reactor or ATR are used to generate superheated steam. The superheated steam may then be used to generate electricity, for example by expanding the superheated steam using a steam turbine with a connected alternator. This arrangement reduces the electricity import to the process.

### Water-gas shift (WGS) section

The WGS section includes one or more WGS shift stages and may include stages of high-temperature shift, medium-temperature shift, isothermal shift and low-temperature shift. These terms, as well as suitable catalysts therefor, are described in WO2022/003312A1.

The stream exiting the WGS unit is referred to as a shifted gas stream and ideally comprises CO₂, H₂, with the residual being unreformed hydrocarbons (predominantly methane), steam and inert gases.

It is preferred that steam and/or water removal is carried out on the shifted gas stream to produce a dewatered shifted stream which is sent to the CO₂ removal section. Typically the shifted stream is cooled to a temperature below the dew point so that the steam condenses. Suitable techniques for steam removal are described in WO2022/003312A1.

### Optional second H₂ membrane separation unit

A second H₂ membrane separation unit may be located downstream from the water-gas shift section and upstream from the CO₂ removal section. The second hydrogen membrane separation unit is arranged to accept the shifted stream, or dewatered shifted stream, and produce a second permeate stream and a second retentate stream, and the CO₂ removal section is arranged to accept said second retentate stream. The terms "second" are used to distinguish these streams from the permeate stream and retentate stream generated by the H₂ membrane separation unit located upstream of the reforming section.

If a second H₂ membrane separation unit is present then it is preferred that the second permeate stream is produced with sufficiently high purity that it can be used as a low carbon fuel directly, without further purification.

### CO₂ removal section

The CO₂ removal section accepts the shifted or dewatered shifted stream, and produces a CO₂ rich stream and a hydrogen product stream. The CO₂ removal section may also accept the permeate stream.

Any suitable CO₂ separation technology may be used and the skilled person will be aware of suitable technologies. In preferred embodiments the CO₂ removal section operates by means of a physical wash system, a reactive wash system or a cryogenic system. A reactive wash system is preferred, more preferably an amine wash system.

As was explained above, it may not be necessary to send the permeate stream for CO₂ removal if it is produced with sufficiently high purity or if it is combined with the purified hydrogen product stream to generate a low carbon fuel stream. It is preferred that the permeate stream is combined with the hydrogen product stream downstream from the CO₂ removal section to form a low carbon fuel stream.

If the permeate stream and the shifted gas stream (or dewatered shifted gas stream) are at suitably similar pressures then the permeate stream and the shifted gas stream may be combined before being fed to the CO₂ removal section. The benefit of this arrangement is that it only requires a single absorber column for the CO₂ removal unit, meaning lower capex and opex.

If the permeate stream and the shifted gas stream (or dewatered shifted gas stream) are at significantly different pressures then it is preferred that the permeate stream and the shifted gas stream are sent to separate CO₂ absorption vessels within the CO₂ removal section, referred to as a permeate CO₂ absorption vessel and a shifted gas CO₂ absorption vessel respectively. The shifted gas CO₂ absorption vessel operates at a higher pressure than the permeate CO₂ absorption vessel. Each absorption vessel may have its own regeneration vessel to desorb CO₂ from the CO₂-laden absorption liquid and generate lean absorption liquid. Alternatively, the CO₂-laden absorption liquid from each vessel may be fed to a common regeneration vessel.

In an embodiment lean absorption liquid from a regeneration vessel is divided proportionally and fed to both the permeate CO₂ absorption vessel and the shifted gas CO₂ absorption vessel, and CO₂-laden absorption liquid from the shifted gas CO₂ absorption vessel is fed to a regeneration vessel, which produces a CO₂ stream and a lean absorption liquid which is recycled to the permeate CO₂ absorption vessel. Circulating the absorption liquid through a single regeneration column is highly efficient. In this arrangement the operating pressure increases between the permeate and shifted gas CO₂ absorption vessels, which achieves more efficient CO₂ removal.

Typically the permeate stream and/or the hydrogen product stream are fed to a fuel gas header, from which the low carbon fuel is withdrawn as required.

The CO₂ rich stream may be further purified if CO₂ is a desired product or may be sent for carbon capture and storage or utilisation. An example of utilisation is in the manufacture of methanol.

### Uses of the low carbon fuel

The permeate stream and/or the hydrogen product stream may be used as a low carbon fuel.

In a preferred embodiment the chemical plant includes one or more burners in which the low carbon fuel is combusted e.g. to heat process stream within or outside of the OTU. It will be appreciated that fired heaters which originally combusted a hydrocarbon-containing fuel may need to be adapted to combust the low carbon fuel.

If the chemical plant comprises a steam methane reformer then it is preferred that the low carbon fuel is used as fuel for the steam methane reformer.

In one embodiment the chemical plant includes a methanol synthesis section fed with a make-up gas stream. The permeate stream and/or the hydrogen product stream may be used to adjust hydrogen content of the make-up gas stream. A portion of the reformed gas stream may be used to produce the make-up gas stream.

The low carbon fuel is typically handled in a fuel gas header at relatively low pressure e.g. 1-10 bara, such as 2-8 bara. This pressure is generally lower than the pressure at which the low carbon fuel stream is produced by the CO₂ removal section. It is therefore preferred that the low carbon fuel is depressurized and used to pressurize other streams, for example off-gas streams or the hydrocarbon-containing fuel stream. This may be achieved for instance by a combined gas expander, motor/generator and compressor.

### Retrofit method

The above sections describe the arrangement of a chemical plant containing an OTU according to the invention. The retrofit method involves installing an OTU arranged as described above, such that a hydrocarbon-containing off-gas stream is converted into a low carbon fuel. The low carbon fuel may be used as fuel in a burner, e.g. to produce steam. It will be appreciated that the original burner may need to be replaced to be suitable for burning the low carbon fuel having a high H₂ content and the method may include installation of H₂ fuel burners for the low carbon fuel, and any other necessary modifications.

### Example

The arrangement shown in Figure 4 was modelled; some heat integration has been omitted from Figure 4 for simplicity. The composition, temperature and pressure of various key streams is shown in Table 1.

**Table 1.**

| | 401 | 403 | 404 | 406 | 409 | 411 | 415 | 418 | 421 | 425 | 426 |
|---|---|---|---|---|---|---|---|---|---|---|---|
| Temperature (C) | 39 | 36 | 35 | 450 | 380 | 198 | 63 | 45.0 | 102 | 10 | 243 |
| Pressure (bar a) | 77.6 | 75.6 | 19.0 | 26.0 | 24.1 | 21.2 | 18.6 | 6.0 | 27.0 | 29.8 | 28.8 |
| Mass flow (te/h) | 22.5 | 14.8 | 7.7 | 68.8 | 101.1 | 150.8 | 87.5 | 18.8 | 3.2 | 16.0 | 32.4 |
| Composition (mol%) | | | | | | | | | | | |
| Water | 0.1 | 0.0 | 0.2 | 42.8 | 28.3 | 35.7 | 1.3 | 0.4 | 0.2 | - | 2.0 |
| Hydrogen | 81.7 | 57.9 | 96.5 | 21.0 | 48.1 | 47.1 | 72.1 | 97.7 | 29.9 | 2.2 | 0.0 |
| Carbon monoxide | 1.7 | 4.2 | 0.2 | 1.6 | 16.8 | 0.1 | 0.2 | 0.2 | 1.3 | - | 0.0 |
| Carbon dioxide | 2.9 | 4.1 | 2.1 | 3.1 | 5.5 | 16.1 | 24.6 | 0.0 | 47.8 | 2.0 | 0.0 |
| Nitrogen | 0.4 | 1.0 | - | 0.5 | 0.4 | 0.3 | 0.5 | 0.5 | 0.5 | 0.9 | 0.9 |
| Argon | - | - | - | - | 0.5 | 0.4 | 0.6 | 0.6 | 18.1 | - | 4.0 |
| Methane | 13.2 | 32.8 | 1.0 | 29.2 | 0.3 | 0.3 | 0.4 | 0.7 | | 87.0 | - |
| Ethane | - | - | - | 1.4 | - | - | - | - | | 6.8 | - |
| Propane | - | - | - | 0.2 | - | - | - | - | | 1.0 | - |
| Butane | - | - | - | - | - | - | - | - | - | 0.1 | |
| Methanol | - | - | - | 0.3 | - | - | 0.3 | - | 1.9 | - | - |
| Oxygen | - | - | - | - | - | - | - | - | | - | 93.1 |
| Lights | - | - | - | - | - | - | - | - | 0.2 | - | - |

The arrangement shown in Figure 4 provides a total CO₂ capture of 1694.5 kmol/h, corresponding to a 96.2% carbon capture rate.

## Claims

1. A chemical plant comprising an off-gas treatment unit arranged to accept a hydrocarbon-containing fuel stream comprising hydrocarbons, steam, and hydrogen, the off-gas treatment unit comprising:
(i) a hydrogen membrane separation unit arranged to accept the hydrocarbon-containing fuel stream and produce a permeate stream and a retentate stream;
(ii) a reforming section comprising a partial oxidation reactor or an autothermal reformer, arranged to accept the retentate stream and produce a reformed gas stream;
(iii) a water-gas shift section arranged to accept said reformed gas stream and produce a shifted gas stream;
(iv) a CO₂ removal section comprising one or more CO₂ removal units, the CO₂ removal section arranged to accept said shifted gas stream and produce a CO₂ rich stream and a hydrogen product stream;
wherein at least a portion of the permeate stream and/or the hydrogen product stream is used as a low carbon fuel.

2. A chemical plant according to claim 1, wherein the hydrocarbon-containing fuel stream is made up from one or more off-gas streams and/or one or more purge streams.

3. A chemical plant according to claim 1, wherein the hydrocarbon-containing fuel stream is produced using a purge stream from a methanol loop.

4. A chemical plant according to any of claims 1 to 3, wherein the retentate stream is combined with a supplementary hydrocarbon-containing stream before being fed to the reforming section.

5. A chemical plant according to any of claims 1 to 4, wherein the reforming section consists essentially of an autothermal reformer.

6. A chemical plant according to any of claims 1 to 5, wherein steam and/or water removal is carried out on the shifted gas stream to produce a dewatered shifted stream which is sent to the CO₂ removal section.

7. A chemical plant according to any of claims 1 to 6, wherein the permeate stream and the shifted gas stream are combined before being fed to the CO₂ removal section.

8. A chemical plant according to any of claims 1 to 6, wherein the permeate stream is fed to a permeate CO₂ absorption vessel and the shifted gas stream is fed to a shifted gas CO₂ absorption vessel, where the shifted gas CO₂ absorption vessel operates at a higher pressure than the permeate CO₂ absorption vessel.

9. A chemical plant according to any of claims 1 to 8, wherein the chemical plant includes a methanol synthesis section fed with a make-up gas stream, and wherein the permeate stream and/or the hydrogen product stream is used to adjust hydrogen content of the make-up gas stream.

10. A chemical plant according to any of claims 1 to 9, wherein the plant is a methanol plant comprising a methanol synthesis section fed with a make-up gas stream, and a portion of the reformed gas stream is used to produce the make-up gas stream.

11. A chemical plant according to any of claims 1 to 10, wherein the permeate stream and/or the hydrogen product stream is used as fuel for a steam methane reformer.

12. A chemical plant according to any of claims 1 to 11, wherein the chemical plant includes one or more burners in which the permeate stream and/or the hydrogen product stream is combusted.

13. A process for converting a hydrocarbon-containing fuel stream into a low carbon fuel, wherein the process is carried out in an off-gas treatment unit as defined in any of claims 1 to 12.

14. A method for retrofitting a chemical plant, comprising the step of installing an off-gas treatment unit arranged to accept a hydrocarbon-containing fuel stream and generate a low carbon fuel, the off-gas treatment unit comprising:
(i) a hydrogen membrane separation unit arranged to accept the hydrocarbon containing fuel stream and produce a permeate stream and a retentate stream;
(ii) a reforming section comprising a partial oxidation reactor or an autothermal reformer, arranged to accept the retentate stream and produce a reformed gas stream;
(iii) a water-gas shift section arranged to accept said reformed gas stream and produce a shifted gas stream;
(iv) a CO₂ removal section comprising one or more CO₂ removal units, the CO₂ removal section arranged to accept said permeate stream and said shifted gas stream and produce a CO₂ rich stream and a hydrogen product stream.

15. A method according to claim 14, wherein post-retrofit the chemical plant is as defined in any of claims 1 to 12.
